# EUROPÄISCHE PATENTSCHRIFT

(11) **EP 4 031 610 B1**
(45) Veröffentlichungstag und Bekanntmachung des Hinweises auf die Patenterteilung: **06.11.2024**
(21) Anmeldenummer: 20771320.7
(22) Anmeldetag: 17.09.2020
(51) Int. Cl.: C08J 11/12, C07C 4/22

(54) **VERFAHREN ZUR DEPOLYMERISATION VON POLYSTYROL**
PROCESS FOR THE DEPOLYMERISATION OF POLYSTRENE
PROCÉDÉ DE DÉPOLYMÉRISATION DE POLYSTYRÈNE

(30) Priorität: 18.09.2019 EP 19197959; 10.01.2020 EP 20000009
(43) Veröffentlichungstag der Anmeldung: 27.07.2022
(73) Patentinhaber: INEOS Styrolution Group GmbH, 60325 Frankfurt (DE)
(72) Erfinder: NIESSNER, Norbert, 67159 Friedelsheim (DE); WILHELMUS, Bianca, 63456 Hanau (DE); SCHMIDT-RODENKIRCHEN, Achim, 95445 Bayreuth (DE); MIERDEL, Konstantin, 95444 Bayreuth (DE); NEUNER, Frank, 95448 Bayreuth (DE)
(74) Vertreter: Jacobi, Markus Alexander
(86) Internationale Anmeldenummer: PCT/EP2020/075984
(87) Internationale Veröffentlichungsnummer: WO 2021/053074

(56) Entgegenhaltungen:
- WO-A1-2018/018153
- US-A- 5 672 794
- YIRONG LIU ET AL: "Pyrolysis of polystyrene waste in a fluidized-bed reactor to obtain styrene monomer and gasoline fraction", FUEL PROCESSING TECHNOLOGY., vol. 63, no. 1, 1 March 2000 (2000-03-01), NL, pages 45 - 55, XP055746249, ISSN: 0378-3820, DOI: 10.1016/S0378-3820(99)00066-1
- GABRIELA DE LA PUENTE ET AL: "Recycled Plastics in FCC Feedstocks: Specific Contributions", INDUSTRIAL & ENGINEERING CHEMISTRY RESEARCH, vol. 36, no. 11, 1 November 1997 (1997-11-01), pages 4530 - 4534, XP055746280, ISSN: 0888-5885, DOI: 10.1021/ie970142a
- DEMENT'EV KONSTANTIN I ET AL: "Thermal depolymerization of polystyrene in highly aromatic hydrocarbon medium", JOURNAL OF ANALYTICAL AND APPLIED PYROLYSIS, ELSEVIER BV, NL, vol. 142, 5 May 2019 (2019-05-05), XP085795141, ISSN: 0165-2370, [retrieved on 20190505], DOI: 10.1016/J.JAAP.2019.05.001
- SAMIH SAID ET AL: "From complex feedstocks to new processes: The role of the newly developed micro-reactors", CHEMICAL ENGINEERING AND PROCESSING: PROCESS INTENSIFICATION, ELSEVIER SEQUOIA, LAUSANNE, CH, vol. 131, 7 July 2018 (2018-07-07), pages 92 - 105, XP085454186, ISSN: 0255-2701, DOI: 10.1016/J.CEP.2018.07.001

## Beschreibung

Die Erfindung betrifft ein Verfahren zur Herstellung von Styrol-Monomeren durch Depolymerisation von Polystyrol, eine Vorrichtung zur Durchführung des Verfahrens sowie die Verwendung einer Vorrichtung zur Depolymerisation (Abbau) von Polystyrol.

Polystyrol ist vorzugsweise ausgewählt aus der Gruppe bestehend aus: Standardpolystyrol (GPPS), schlagzähem Polystyrol (HIPS), Styrol-Butadien-Copolymeren. Besonders bevorzugt sind schlagzähes Polystyrol (HIPS) und / oder Standardpolystyrol. Schlagzähe Polystyrole (HIPS) und Standardpolystyrole, sowie deren Herstellung, Struktur und Eigenschaften sind in der Übersichtsliteratur ausführlich beschrieben (A. Echte, F. Haaf, J. Hambrecht in Angew . Chem. (Int. Ed. Engl.) 20, 344-361 (1981); und auch im Kunststoffhandbuch, herausgegeben von R. Vieweg und G. Daumiller, Band 4 "Polystyrol", Carl-Hanser-Verlag München (1996).

Der Übergang von einer Linear- zu einer Kreislaufwirtschaft ist angesichts von Klimawandel, Umweltverschmutzung, Bevölkerungswachstum und Ressourcenabhängigkeit sowohl ökologisch als auch ökonomisch erforderlich. In den 1980er und 1990er Jahren wurden bereits intensive Anstrengungen zur Entwicklung von Verfahren für die rohstoffliche Wiederverwertung von Kunststoffabfällen unternommen, wobei es aufgrund ungelöster verfahrenstechnischer Problemstellungen und aus wirtschaftlichen Gründen bisher nicht zu großtechnischen Anwendungen kam. Aufgrund des allgemein wachsenden Umweltbewusstseins und einem steigenden Bedarf an nachhaltigen Lösungen wächst allerdings auch das Interesse an chemischem Recycling.

Nicht alle thermoplastischen Polymere sind gleichermaßen für das chemische Recycling geeignet. Bei der thermischen Zersetzung von Polyolefinen oder Polyestern entstehen Gemische aus u. a. Wachsen, Leichtöl und Gasen. Der Abbau von Polyethylenterephthalat (PET) führt zu organischen Säuren, hauptsächlich Benzoesäure und Terephthalsäure, die korrosiv sind, und auch eine Verstopfung des Reaktors verursachen können (G. Grause et al., Rohstoffrecycling von polymerem Abfallmaterial, in: Journal of Material Cycles and Waste Management, 13(4), 2011, 265-282).

Im Fall von Polystyrol und anderen Styrol-haltigen Polymeren ist es möglich, diese Polymere in ihre Grundbestandteile, insbesondere Styrol-Monomere, zu depolymerisieren, wodurch Polystyrol und andere Styrol-Monomer-haltige Polymere eine ausgezeichnete Wahl für das chemische Recycling bilden.

Das resultierende Produktgemisch eines Depolymerisationsprozesses muss jedoch gereinigt werden, um die Komponenten als Rohmaterial für neue Zwecke, wie Polymerisationsprozesse, zu verwenden.

Wenn Polystyrol ausreichend thermisch behandelt wird, zersetzt es sich zu Styrol-Monomeren, aber eine unvollständige Zersetzung führt auch zur Bildung von z.B. Styrol-Dimeren, -Trimeren und anderen Oligomeren. Wenn die Zersetzungsbedingungen zu harsch sind, können Nebenprodukte wie Benzol, Toluol, Ethylbenzol, Cumol und alpha-Methylstyrol gebildet werden. Die Mengen dieser Reaktionsprodukte variieren und hängen von den Reaktionsbedingungen und den verwendeten Rohstoffen ab (s. C. Bouster et al., Study of the pyrolysis of polystyrenes: Kinetics of thermal decomposition, Journal of Analytical and Applied Pyrolysis, 1 (1980) 297-313 und C. Bouster, Evolution of the product yield with temperature and molecular weight in the pyrolysis of polystyrene, in: Journal of Analytical and Applied Pyrolysis 15 (1989) 249-259).

Die gewonnenen Styrol-Monomere können für ein neues Polymerisationsverfahren verwendet werden. Styrol-Oligomere können ggf. den Polymerisationsprozess stören, da sie bereits in geringen Mengen wichtige Eigenschaften des Polymers beeinflussen. Dies gilt auch für andere Nebenprodukte. Daher sollten die Styrol-Monomere von anderen Komponenten des Produktgemisches getrennt werden, um eine hohe Produktqualität zu gewährleisten.

Insbesondere andere aromatische Verbindungen als Styrol-Monomere können bei radikalischen Polymerisationsverfahren als Kettenübertragungsmittel wirken, die das durchschnittliche Molekulargewicht der hergestellten Polymere senken und zu Polymeren mit einer niedrigeren Glasübergangstemperatur (Tg) beitragen (s. DS Achilias et al., Chemical recycling of polystyrene by pyrolysis: Potential use of the liquid product for the reproduction of polymer, in: Macromolecular Materials and Engineering, 292(8) (2007), 923-934). Protonen von z.B. Carbonsäuren, Alkoholen, Aldehyden oder Ketonen wirken als Abbruchmittel bei anionischen Polymerisationsverfahren von Styrol (s. D. Baskaran, Anionic Vinyl Polymerization, in: Controlled and Living Polymerizations: From Mechanisms to Applications, John Wiley & Sons, 2009, 1-56).

Im vorliegenden Verfahren wird Polystyrol, vorzugsweise ausgewählt aus der Gruppe Standardpolystyrol (GPPS), schlagzähem Polystyrol (HIPS) und Styrol-Butadien-Copolymeren eingesetzt. Weiterhin können die verwendeten schlagzähen Polystyrole durch die Verwendung spezieller Polybutadienkautschuke mit beispielsweise einer modifizierten 1,4-cis- und / oder 1,4-trans-Fraktion oder 1,2- und 1,4-Bindung strukturell modifiziert worden sein gegenüber herkömmlichen Kautschuken.

Weiterhin können anstelle von Polybutadienkautschuk auch andere Dienkautschuke sowie Elastomere vom Typ Ethylen-Propylen-Dien-Copolymer (EPDM-Kautschuk) sowie hydrierte Dienkautschuke oder auch Siliconkautschuke eingesetzt werden.

In dem als Polystyrol verwendeten schlagfesten Polystyrol beträgt der Dienkautschukanteil, insbesondere der Polybutadienkautschukanteil, im allgemeinen 5 bis 12 Gew .-%, bevorzugt 6 bis 10 Gew .-%, besonders bevorzugt 7 bis 9 Gew .-% und der Polystyrolanteil beträgt im allgemeinen 88 bis 95 Gew .-%, bevorzugt 90 bis 94 Gew .-%, bevorzugter 91 bis 93 Gew .-%, wobei die Summe aus Polystyrolanteil und Dienkautschukanteil 100 Gew .-% ergibt.

Geeignetes Standardpolystyrol wird nach der Methode der anionischen oder radikalischen Polymerisation hergestellt. Dabei ist die durch den Polymerisationsprozess beeinflussbare Molekulargewchts-Uneinheitlichkeit des Polymers von untergeordneter Bedeutung. Bevorzugt sind Standardpolystyrol und schlagzähes Polystyrol, dessen toluollöslicher Anteil ein mittleres Molekulargewicht Mw von 150.000 bis 300.000 g / mol, besonders bevorzugt 150.000 bis 270.000 g / mol aufweist und das gegebenenfalls weiter ausgestattet ist mit Zusatzstoffen, wie beispielsweise Mineralöl (zB Weißöl), Stabilisator, Antistatika, Flammschutzmittel oder Wachse.

Als Polystyrol eingesetzte Styrol-Copolymere können Methyl(meth)acrylat, α-Methylstyrol und/oder Maleinsäureanhydrid, sowie weitere, copolymerisierbare Monomere enthalten.

In JP 2005132802 und JPH 11100875 (Toshiba Plant Systems) wird ein Verfahren zur Rückgewinnung von Styrol aus Polystyrol-Abfällen beschrieben, jedoch wird keine Lösung zur Trennung von Leichtsiedern, Styrol und Schwersiedern bereitgestellt.

Es besteht ein hoher Bedarf an einem Verfahren zur Herstellung von Styrol-Monomeren durch Depolymerisation von Polystyrol, das Styrol in sehr hoher Ausbeute liefert und dabei die Bildung von Styrol-Oligomeren möglichst gering hält, um den nachfolgenden Reinigungsprozess zu minimieren.

Es ist bekannt, dass das Wirbelschicht-Verfahren zur Depolymerisation von Polymeren wie u.a. Polystyrol geeignet ist. Liu et al. ("Pyrolysis of polystyrene waste in a fluidizedbed reactor to obtain styrene monomer and gasoline fraction", Fuel Processing Technology, 63, 45-55 (2000)) und Williams et al. ("Product composition from the fast pyrolysis of polystyrene", Environmental Technology, 20, 1109-1118 (1999)) untersuchen die Pyrolyse von PS-Abfällen mittels eines Wirbelschichtreaktors und beobachten dabei die Depolymerisation von Polystyrol in Styrol-Mono- und -Oligomere.

US 5 672 794 A, Gabriela de la Puente et al.: "Recycled Plastics in FCC Feedstocks: Specific Contributions" (Industrial & Engineering Chemistry Research 1997, 36, 4530-4534), Dement'ev Konstantin et al: "Thermal depolymerization of polystyrene in highly aromatic hydrocarbon medium" (Journal of Analytical and Applied Pyrolysis 2019, 142), Samih Said et al.: "From complex feedstocks to new processes: The role of the newly developed micro-reactors" (Chemical Engineering andProcessing: Process Intensification 2018, 131, 92-105), und WO 2018/018153 A1 beschreiben auch verschiedene Verfahren zur Depolymerisation von Polystyrol in Wirbelschichtreaktoren und anderen Reaktoren unter verschiedenen Bedingungen.

Eine Aufgabe der Erfindung ist es, ein Verfahren zur Herstellung von Styrol-Monomeren durch Depolymerisation von Polystyrol mit optimierten Prozessbedingungen bereitzustellen, um die Ausbeute an Styrol-Monomeren zu maximieren. Auch sollen schädliche Nebenprodukte vermieden werden.

Es wurde gefunden, dass bestimmte Kombinationen aus Depolymerisations-Temperatur und Verweilzeit im Reaktor zu besonders hohen Styrol-Monomer-Ausbeuten führen.

Gegenstand der Erfindung ist ein Verfahren zur Herstellung von Styrol-Monomeren durch Depolymerisation von Polystyrol, umfassend die Schritte:
a) Zuführen einer Polymerzusammensetzung (A) enthaltend 60 bis 99,9 Gew.-% Polystyrol, bezogen auf das Gesamtgewicht der Polymerzusammensetzung (A), in die Reaktionszone (R) eines Pyrolysereaktors (P);
b) thermisches Spalten des in der Polymerzusammensetzung (A) enthaltenen Polystyrols in der Reaktionszone (R) des Pyrolysereaktors (P) bei einer Temperatur von 450 °C bis 1000 °C, um ein Produktgemisch (G) enthaltend Styrol-Monomere und weitere Komponenten zu erhalten;
c) Entnehmen des in Schritt b) erhaltenen Produktgemisches (G) aus der Reaktionszone (R) des Pyrolysereaktors (P);
d) Abkühlen des in Schritt c) entnommenen Produktgemisches (G), um ein kondensiertes Produktgemisch (K), enthaltend Styrol-Monomere und weitere Komponenten, zu erhalten; und
e) Abtrennen der Styrol-Monomere von den weiteren Komponenten des in Schritt d) erhaltenen kondensierten Produktgemisches (K),
wobei die mittlere Verweilzeit (Z) der Polymerzusammensetzung (A) in der Reaktionszone (R) des Pyrolysereaktors (P) von 0,01 s bis 10 s beträgt;

Hierbei ist der Pyrolysereaktor (P) ein Wirbelschichtreaktor, dessen Reaktionszone (R) eine Siliciumcarbid-Wirbelschicht umfasst, und/oder die Temperatur in der Reaktionszone (R) des Pyrolysereaktors (P) beträgt 580 °C bis 630 °C bei einer mittleren Verweilzeit (Z) der Polymerzusammensetzung (A) in der Reaktionszone des Pyrolysereaktors von 0,4 s bis 0,6 s.

Die Polymerzusammensetzung (A), die in dem erfindungsgemäßen Verfahren in Schritt a) in die Reaktionszone (R) des Pyrolysereaktors (P) zugeführt wird, enthält 60 bis 99,9 Gew.-%, bevorzugt 80 bis 99,9 Gew.-%, besonders bevorzugt 90 bis 99,9 Gew.-%, ganz besonders bevorzugt 95 bis 99,9 Gew.-% Polystyrol, bezogen auf das Gesamtgewicht der Polymerzusammensetzung (A).

Bei den übrigen 0,1 bis 40 Gew.-%, bevorzugt 0,1 bis 20 Gew.-%, besonders bevorzugt 0,1 bis 10 Gew.-%, ganz besonders bevorzugt 0,1 bis 5 Gew.-%, bezogen auf das Gesamtgewicht der Polymerzusammensetzung (A) kann es sich um andere polymere Komponenten als Polystyrol und/oder um Additive und/oder andere Fremdstoffe handeln.

Sind andere Polymere als Polystyrol in der Polymerzusammensetzung (A) enthalten, sind diese beispielsweise ausgewählt aus der Gruppe bestehend aus Polyolefinen, Polyestern, (Co-)polymeren von Acrylnitril, (Co-)polymeren von anderen vinylaromatischen Monomeren als Styrol, (Co-)polymeren von Alkyl(meth)acrylaten und Copolymeren von Styrol mit anderen Monomeren, die nicht zu den anderen hier gelisteten (Co-)polymeren gehören.

Als andere Polymere als Polystyrol geeignet sind beispielsweise Copolymere von vinylaromatischen Monomeren mit weiteren Comonomeren wie Butadien, (Meth)acrylat, Acrylnitril, alpha-Methylstyrol und Phenylmaleinimid. Insbesondere sind dies Vinylaromat-Copolymere ausgewählt aus der Gruppe bestehend aus: Styrol-Acrylnitril-Copolymeren (SAN), α-Methylstyrol-Acrylnitril-Copolymeren (AMSAN), Styrol-Maleinsäureanhydrid-Copolymeren, Styrol-Phenyl-maleinimid-Copolymeren, Styrol-Methylmethacrylat-Copolymeren (SMMA), Styrol-Acrylnitril-Maleinsäureanhydrid-Copolymeren, Styrol-Acrylnitril-Phenylmaleinimid-Copolymeren, α-Methylstyrol-Acrylnitril-Methylmethacrylat-Copolymeren, α-Methylstyrol-Acrylnitril-t-Butylmethacrylat-Copolymeren, Styrol-Acrylnitril-t-Butylmethacrylat-Copolymeren.

Ebenfalls geeignete andere Polymere sind beispielsweise Dien-Kautschuke wie Homopolymere von konjugierten Dienen und Copolymere solcher Diene untereinander, Copolymere solcher Diene mit Acrylaten, insbesondere n-Butylacrylat, und Copolymere solcher Diene mit den Comonomeren ausgewählt aus Styrol, α-Methylstyrol, Acrylnitril, Methacrylnitril, Methylmethacrylat (MMA), Maleinsäureanhydrid (MSA) und N-Phenylmaleinimid (N-PMI). Die Dien-Kautschuke können auch vernetzend wirkende, polyfunktionelle Monomere enthalten.

Ebenfalls geeignet sind Polyolefine wie PE-LD (Low Density Polyethylen), PE-LLD (Linear Low Density Polyethylen), PE-HD (High Density Polyethylen), Metallocen-Polyethylene, Ethylen-Copolymere wie Poly(ethylen-co-vinylacetat), Ethylen-Buten, Ethylen-Hexen, Ethylen-Octen-Copolymere, sowie Cycloolefincopolymere einzeln oder als Mischung. Darüber hinaus kommen beispielsweise Polypropylen, wie Homo- oder Copolymere des Propylens, Metallocen-katalysierte Polypropylene sowie Copolymere des Propylens mit anderen, dem Fachmann bekannten Comonomeren in Frage.

Polycarbonate, Polyamide, Poly(meth)acrylate, Polyvinylchloride, Polyester, halogenierte Polymere, Vinylaromat-Dien-Copolymere (SBC), Polyether, Polysulfone, Polyethersulfone, Polyimidazole und verwandten Polymere können ebenfalls in der Polymerzusammensetzung (A) enthalten sein.

Bevorzugt enthält die Polymerzusammensetzung (A) weniger als 30 Gew.-%, besonders bevorzugt weniger als 15 Gew.-%, ganz besonders bevorzugt weniger als 5 Gew.-%, bezogen auf das Gesamtgewicht der Polymerzusammensetzung (A), an Polyolefinen, weniger als 5 Gew.-%, besonders bevorzugt weniger als 2 Gew.-%, ganz besonders bevorzugt weniger als 1 Gew.-%, bezogen auf das Gesamtgewicht der Polymerzusammensetzung (A), an (Co-)polymeren von Acrylnitril, und weniger als 5 Gew.-%, besonders bevorzugt weniger als 2 Gew.-%, ganz besonders bevorzugt weniger als 1 Gew.-%, bezogen auf das Gesamtgewicht der Polymerzusammensetzung (A), an Polyestern.

Vorzugsweise enthält die Polymerzusammensetzung (A) keine anderen polymeren Komponenten als Polystyrol.

Als Additive in der Polymerzusammensetzung (A) können 0 bis 20 Gew.-%, bezogen auf das Gesamtgewicht der Polymerzusammensetzung (A), übliche Kunststoffadditive und -Hilfsstoffe enthalten sein. Beispielshaft kann ein Additiv oder ein Hilfsstoff ausgewählt sein aus der Gruppe bestehend aus Antioxidantien, UV-Stabilisatoren, Peroxidzerstörern, Antistatika, Gleitmitteln, Entformungsmitteln, Flammschutzmitteln, Füll- oder Verstärkerstoffen (Glasfasern, Kohlefasern, etc.), Farbmitteln und Kombinationen aus zwei oder mehr daraus. Als Beispiele für Oxidationsverzögerer und Wärmestabilisatoren seien Halogenide von Metallen der Gruppe I des periodischen Systems, z.B. Natrium-, Kalium- und/oder Lithiumhalogenide, ggf. in Verbindung mit Kupfer-(I)-Halogeniden, z.B. Chloriden, Bromiden, Jodiden, sterisch gehinderte Phenole, Hydrochinone, verschiedene substituierte Vertreter dieser Gruppen und deren Mischungen in Konzentrationen bis zu 1 Gew.-%, bezogen auf das Gesamtgewicht der Polymerzusammensetzung (A) genannt.

Als UV-Stabilisatoren, die im Allgemeinen in Mengen bis zu 2 Gew.-%, bezogen auf das Gesamtgewicht der Polymerzusammensetzung (A) enthalten sind, seien verschiedene substituierte Resorcine, Salicylate, Benzotriazole und Benzophenone genannt.

Weiterhin können organische Farbstoffe wie Nigrosin, Pigmente wie Titandioxid, Phthalocyanine, Ultramarinblau und Ruß als Farbstoffe in der Polymerzusammensetzung (A) enthalten sein, sowie faser- und pulverförmige Füllstoffe und Verstärkungsmittel. Beispiele für letztere sind Kohlenstoffasern, Glasfasern, amorphe Kieselsäure, Calciumsilicat (Wollastonit), Aluminiumsilicat, Magnesiumcarbonat, Kaolin, Kreide, gepulverter Quarz, Glimmer und Feldspat.

Als Keimbildungsmittel können z.B. Talkum, Calciumfluorid, Natriumphenylphosphinat, Aluminiumoxid, Siliciumdioxid und Nylon 22 enthalten sein.

Gleit- und Entformungsmittel, welche in der Regel in Mengen bis zu 1 Gew.-%, bezogen auf das Gesamtgewicht der Polymerzusammensetzung (A), eingesetzt werden können, sind z.B. langkettige Fettsäuren wie Stearinsäure oder Behensäure, deren Salze (z.B. Ca- oder Zn-Stearat) oder Ester (z.B. Stearylstearat oder Pentaerythrittetrastearat) sowie Amidderivate (z.B. Ethylenbisstearylamid).

Weiterhin können Antiblockmittel auf mineralischer Basis in Mengen bis zu 0,1 Gew.-%, bezogen auf das Gesamtgewicht der Polymerzusammensetzung (A), enthalten sein. Als Beispiele seien amorphe oder kristalline Kieselsäure, Calciumcarbonat oder Aluminiumsilikat genannt.

Als Verarbeitungshilfsmittel kann beispielsweise Mineralöl, vorzugsweise medizinisches Weißöl, in Mengen bis zu 5 Gew.-%, vorzugsweise bis zu 2 Gew.-%, bezogen auf das Gesamtgewicht der Polymerzusammensetzung (A), enthalten sein.

Als Beispiele für Weichmacher seien Phthalsäuredioctylester, Phthalsäuredibenzylester, Phthalsäurebutylbenzylester, Kohlenwasserstofföle, N-(n-Butyl)benzolsulfonamid und o- und p-Tolylethylsulfonamid genannt.

Weiterhin können alle für die jeweiligen Thermoplaste bekannten Flammschutzmittel enthalten sein, insbesondere solche auf Basis von Phosphorverbindungen oder roter Phosphor selbst.

Weiterhin können noch Feuchtigkeit und/oder weitere anorganische und/oder organische Fremdbestandteile, wie z.B. Nahrungsmittelrückstände, enthalten sein.

In einer besonders bevorzugten Ausführungsform besteht die Polymerzusammensetzung (A) aus 90 bis 99,9 Gew.-%, bezogen auf das Gesamtgewicht der Polymerzusammensetzung (A), an Polystyrol und 0,1 bis 10 Gew.-%, bezogen auf das Gesamtgewicht der Polymerzusammensetzung (A), an Additive(n). In einer ganz besonders bevorzugten Ausführungsform besteht die Polymerzusammensetzung (A) aus 95 bis 99,9 Gew.-%, bezogen auf das Gesamtgewicht der Polymerzusammensetzung (A), Polystyrol und 0,1 bis 5 Gew.-%, bezogen auf das Gesamtgewicht der Polymerzusammensetzung (A), Additiv(en).

Bevorzugt besteht die Polystyrol-Komponente, die in der Polymerzusammensetzung (A) enthalten ist, im Wesentlichen, vorzugsweise vollständig, aus GPPS (general purpose polystyrene). In diesem Zusammenhang bedeutet "im Wesentlichen", dass in der Polystyrol-Komponente keine Bestandteile enthalten sind, die die Eigenschaften des GPPS in signifikanter Weise verändern.

Die erfindungsgemäß eingesetzte Polymerzusammensetzung (A) kann gegebenenfalls in geeigneter Weise vorbehandelt werden, um beispielsweise anhaftende Verunreinigungen wie etwa Lebensmittelreste oder Schmutz, Feuchtigkeit und Fremdstoffe wie Metalle oder andere Stoffe und Verbundmaterialien zu entfernen.

Dies erfolgt vorteilhaft in einer Vorbehandlung, die einen oder mehrere der folgenden Schritte umfassen kann, wobei die Reihenfolge der Schritte nicht festgelegt ist und Schritte auch mehrfach wiederholt werden können: manuelle Störstoffsortierung, Wäsche, Zerkleinerung, automatische Sortierung in geeigneten Anlagen. Gegebenenfalls können auch Polymerzusammensetzungen, die der Polymerzusammensetzung (A) nicht entsprechen, durch ein solches Verfahren in eine erfindungsgemäß eingesetzte Polymerzusammensetzung (A) umgewandelt werden.

Bevorzugt ist daher auch eine Ausführungsform des erfindungsgemäßen Verfahrens, bei der eine Polymerzusammensetzung, die der Polymerzusammensetzung (A) nicht entspricht, einer Vorbehandlung unterworfen wird, die einen oder mehrere der folgenden Schritte umfasst, wobei die Reihenfolge der Schritte nicht festgelegt ist und Schritte auch mehrfach wiederholt werden können: manuelle Störstoffsortierung, Wäsche, Zerkleinerung, automatische Sortierung in geeigneten Anlagen, um so die Polymerzusammensetzung (A) zu erhalten.

Die thermische Zersetzung des Polystyrols wird in einem Wirbelschichtreaktor mit einer Siliciumcarbid-Wirbelschicht in der Reaktionszone (R) durchgeführt. Alternativ kann die Zersetzung des Polystyrols bei einer Temperatur von 580 °C bis 630 °C und einer mittleren Verweilzeit (Z) der Polymerzusammensetzung von 0,4 s bis 0,6 s in jedem geeigneten Reaktor stattfinden, in dem die zur Zersetzung erforderliche Temperatur erreicht werden kann. Beispielsweise kann die thermische Zersetzung in einem Drehrohrofen durchgeführt werden. Drehrohröfen sind z.B. in EP-A 1481957 beschrieben. Die thermische Zersetzung kann auch in Extrudern stattfinden, diese sind z.B. in EP-A 1966291 beschrieben. Solche Reaktoren können mit oder ohne Gasstrom betrieben werden, etwa Trägergas oder Gas als Reaktionsmedium.

Der Pyrolysereaktor (P) kann demnach im Prinzip eine beliebige bekannte Art von Pyrolysereaktor sein, unter der Voraussetzung, dass der Aufbau des Pyrolysereaktors eine genaue Einstellung der Temperatur in der Reaktionszone (R) des Pyrolysereaktors (P) und eine genaue Einstellung der mittleren Verweilzeit (Z) der Polymerzusammensetzung (A) in der Reaktionszone (R) des Pyrolysereaktors (P) ermöglicht.

In diesem Zusammenhang bedeutet "genaue Einstellung", dass die Abweichung der tatsächlichen Temperatur in der Reaktionszone (R) des Pyrolysereaktors (P) von der eingestellten Temperatur höchstens 10 °C, bevorzugt höchstens 5 °C, besonders bevorzugt höchstens 1 °C beträgt, bzw. dass die Abweichung der tatsächlichen mittleren Verweilzeit (Z) von der eingestellten mittleren Verweilzeit (Z) höchstens 0,2 s, bevorzugt höchstens 0,1 s, besonders bevorzugt höchstens 0,05 s, ganz besonders bevorzugt höchstens 0,01 s beträgt.

Bei dem Pyrolysereaktor (P) handelt es sich vorzugsweise um einen Wirbelschichtreaktor, beschrieben z.B. in Schildhauer et al., "Reaktoren für Fluid-Feststoff-Reaktionen: Wirbelschichtreaktoren" In: Reschetilowski W. (Hrsg.): Handbuch Chemische Reaktoren, Springer Spektrum, Berlin, Heidelberg (2019). Üblicherweise wird dabei ein Gas oder ein Gasgemisch als Fluidisierungs-Medium verwendet. Das Gas oder Gasgemisch ist üblicherweise nicht-reaktiv bei den im Pyrolysereaktor (P) vorherrschenden Bedingungen. Bevorzugt wird das Gas ausgewählt aus Wasserdampf, Stickstoff, Edelgasen (Xe, Ar, Ne, He) oder einer Mischung daraus.

Das Gas oder Gasgemisch kann als separater Gasstrom in den Pyrolysereaktor (P) eingebracht werden. Die optimale Fließgeschwindigkeit des Gases oder Gasgemisches hängt von der Reaktorkonfiguration sowie von den Reaktions- oder Prozessbedingungen ab.

Die Optimierung des Prozesses ist über die Parameter Temperatur, Verweilzeit des Reaktionsgemisches in der Reaktionszone (R) des Pyrolysereaktors (P) und Konzentration der Ausgangsstoffe möglich. Der Bereich des Reaktors, in dem das Reaktionsgemisch solchen Bedingungen unterworfen wird, bei denen Depolymerisations-Reaktionen stattfinden, wird hier als Reaktionszone (R) bezeichnet.

Der Pyrolysereaktor (P) kann ein festes Medium enthalten, z.B. SiOz (Quarzsand) oder SiC. In einer bevorzugten Ausführungsform ist der Pyrolysereaktor (P) ein Wirbelschichtreaktor, der in der Reaktionszone (R) eine Wirbelschicht aus Siliciumcarbid (SiC) umfasst. SiC verfügt, neben hoher chemischer und mechanischer Stabilität, über eine gute Wärmeleitung und Wärmekapazität. Diese Kombination ist für die Durchführung endothermer Prozesse vorteilhaft.

Bevorzugt wird SiC mit einer gewichtsmittleren Partikelgröße von 20 bis 1000 µm, besonders bevorzugt 40 bis 500 µm verwendet. Bei solchen Partikelgrößen wird eine besonders gute Fluidisierung des Wirbelbettes erreicht und eine besonders effektive Wärmeübertragung auf die Polymerzusammensetzung (A) ermöglicht.

Es wurde festgestellt, dass die Ausbeute an Styrol-Monomeren besonders hoch ist, wenn die Verweilzeit des Polystyrols in der Depolymerisations-Zone kurz gewählt wird. Dies kann in Wirbelschichtreaktoren oder anderen Reaktoren erreicht werden, die einen Gasstrom zum Transport der eingeführten Polymerzusammensetzung (A) durch den Reaktor nutzen. Die mittleren Verweilzeiten des Reaktionsgutes in einem Wirbelschichtreaktor mit einer Siliciumcarbid-Wirbelschicht in der Reaktionszone (R) liegen zwischen 0,1 Sekunden und 10 Sekunden. Kurze Verweilzeiten können durch hohe Fließgeschwindigkeiten des Gasstromes erreicht werden. Die Depolymerisation kann bei Drücken von 0,01 bar bis 5 bar oder bei Atmosphärendruck (1 bar) durchgeführt werden. Bevorzugt wird der Gasstrom vorgeheizt, bevor er in die Reaktionszone (R) geleitet wird. Das Vorheizen erfolgt bevorzugt bei einer Temperatur, die von der Temperatur in der Reaktionszone (R) des Pyrolysereaktors (P) um höchstens 200 °C nach unten abweicht.

Die Temperatur in der Reaktionszone (R) des Pyrolysereaktors (P) wird in dem erfindungsgemäßen Verfahren in einem Wirbelschichtreaktor mit einer Siliciumcarbid-Wirbelschicht in der Reaktionszone (R) auf 400 bis 1000 °C, oftmals 450 bis 1000 °C, bevorzugt auf 500 bis 800 °C, besonders bevorzugt auf 550 bis 650 °C, ganz besonders bevorzugt auf 580 bis 630 °C, und die mittlere Verweilzeit der Polymerzusammensetzung (A) in der Reaktionszone (R) des Pyrolysereaktors (P) auf 0,1 bis 10 s, bevorzugt auf 0,2 bis 2 s, besonders bevorzugt auf 0,3 bis 1 s, ganz besonders bevorzugt auf 0,4 bis 0,6 s eingestellt. In einer bevorzugten Ausführungsform ist die Temperatur in der Reaktionszone (R) 500 bis 800 °C und die mittlere Verweilzeit der Polymerzusammensetzung (A) darin 0,2 bis 2 s. In einer besonders bevorzugten Ausführungsform ist die Temperatur in der Reaktionszone (R) 550 bis 650 °C und die mittlere Verweilzeit der Polymerzusammensetzung (A) darin 0,3 bis 1 s. In einer ganz besonders bevorzugten Ausführungsform ist die Temperatur in der Reaktionszone (R) 580 bis 630 °C und die mittlere Verweilzeit der Polymerzusammensetzung (A) darin 0,4 bis 0,6 s.

Die Einstellung der Temperatur kann auf beliebige bekannte Art erfolgen, beispielsweise durch Mikrowelleneinstrahlung, mit Hilfe von Wärmetauschern, Gasbrennern, resistiven Heizleitern (Widerstandsheizung), oder durch Einleiten von überhitztem Gas, insbesondere Wasserdampf, jeweils allein oder in Kombination. In einer Ausführungsform erfolgt die Einstellung der Temperatur mit Hilfe von resistiven Heizleitern, die mit der Wand der Reaktionszone (R) des Pyrolysereaktors (P) in Kontakt stehen. Alternativ kann die Einstellung der Temperatur mit Hilfe von Wasserdampf erfolgen, der durch Verdampfen von Wasser zur Verfügung gestellt und durch einen Dampfüberhitzer auf die gewünschte Temperatur gebracht wird. In einer Ausführungsform wird eine Kombination von resistiven Heizleitern, die mit der Wand der Reaktionszone (R) des Pyrolysereaktors (P) in Kontakt stehen, und Wasserdampf für die Einstellung der Temperatur in der Reaktionszone (R) des Pyrolysereaktors (P) verwendet.

In einer bevorzugten Ausführungsform ist der Pyrolysereaktor (P) ein Wirbelschichtreaktor, vorzugsweise umfassend eine SiC-Wirbelschicht in dessen Reaktionszone (R), wobei die Temperatur in der Reaktionszone (R) durch Einleiten von Wasserdampf der gewünschten Temperatur eingestellt wird. In dieser Ausführungsform wird der Wasserdampf durch einen Verdampfer zur Verfügung gestellt und über einen Dampfüberhitzer auf die gewünschte Temperatur gebracht. Ebenfalls wird in dieser Ausführungsform der Wasserdampf sowohl zum Einstellen der Temperatur in der Reaktionszone (R) als auch zur Erzeugung der Wirbelschicht verwendet. Optional kann bei dieser Ausführungsform die Heizenergie in der Reaktionszone (R) zusätzlich durch resistive Heizleiter, die mit der Wand der Reaktionszone (R) des Pyrolysereaktors (P) in Kontakt stehen, zur Verfügung gestellt werden.

Die Teilchengröße und die Konzentration der Polymerzusammensetzung (A) in der Reaktionszone (R) können an die spezifischen Gegebenheiten des Reaktors und seiner Konfiguration, an die Reaktionsbedingungen und an die Zusammensetzung der Polymerzusammensetzung (A) angepasst werden. Typischerweise hat die Polymerzusammensetzung (A) eine Teilchengröße zwischen 100 µm und 50 mm, bevorzugt zwischen 250 µm und 5 mm, besonders bevorzugt zwischen 500 µm und 2 mm. Die Teilchen können eine sphärische oder eine nicht-sphärische Form aufweisen. Im Fall einer sphärischen Form wird die Partikelgröße durch Messung des volumenmittleren Durchmessers bestimmt. Im Fall von nicht-sphärischen Partikeln, beispielsweise nadelförmigen Partikeln, wird die längste Dimension zur Bestimmung der Partikelgröße herangezogen.

Die Konzentration des Reaktionsgutes im Reaktor hängt vom Reaktortyp, der Reaktorgröße und der Reaktorkonfiguration ab. Typischerweise liegt die Konzentration der Polymerzusammensetzung (A) in der Reaktionszone (R) des Pyrolysereaktors (P) zwischen 1 und 50 Gew.-%, bevorzugt zwischen 1 und 25 Gew.-%, besonders bevorzugt zwischen 1 und 10 Gew.-%, ganz besonders bevorzugt zwischen 2 und 7 Gew.-%.

Bevorzugt wird die Polymerzusammensetzung (A) in Schritt a) pneumatisch in die Reaktionszone (R) des Pyrolysereaktors (P) zugeführt, mit der Wirbelschicht aus Siliciumcarbid vermischt und schließlich in Schritt b) depolymerisiert. Besonders bevorzugt erfolgt die Zuführung der Polymerzusammensetzung (A) in Schritt a) kontinuierlich.

Während der Verweilzeit (Z) in der Reaktionszone (R) des Pyrolysereaktors (P) wird die Polystyrol-Komponente und gegebenenfalls andere Polymere der Polymerzusammensetzung (A) zumindest teilweise depolymerisiert, um ein Produktgemisch (G) enthaltend Styrol-Monomere und andere Komponenten zu ergeben.

Das Produktgemisch (G), enthaltend Styrol-Monomere und andere Komponenten wird in Schritt c) aus der Reaktionszone (R) des Pyrolysereaktors (P) entnommen. Bevorzugt findet die Entnahme kontinuierlich statt. Besonders bevorzugt wird das Produktgemisch (G) in gasförmigem Zustand aus dem oberen Bereich der Reaktionszone (R) des Pyrolysereaktors (P) kontinuierlich entnommen. Hierbei kann die Entnahme des Produktgemisches (G) beispielsweise durch Beförderung des Produktgemisches (G) aus der Reaktionszone (R) aufgrund des reaktionsbedingt erhöhten Drucks in der Reaktionszone (R) automatisch erfolgen.

Der Pyrolysereaktor kann eine Quensche enthalten oder mit einer Quensche verbunden sein. Unter Quensche wird in diesem Zusammenhang ein Bereich des Pyrolysereaktors verstanden, in dem die Depolymerisationsreaktion schnell abgestoppt wird, bevorzugt durch Abkühlen des heißen Gases bestehend aus dem Produktgemisch (G) und inertem Gasstrom, beispielsweise Wasserdampf. Die Quensche dient unter anderem zur Stabilisierung der Reaktionsprodukte und zur Verhinderung oder Verminderung einer unerwünschten Repolymerisation. Typische Quenschen kühlen das Produktgemisch (G) von einer Temperatur von mehr als 400 °C auf eine Temperatur von unter 350 °C innerhalb eines sehr kurzen Zeitraums ab, bevorzugt innerhalb von weniger als 10 Sekunden, besonders bevorzugt innerhalb von weniger als 5 Sekunden, besonders bevorzugt innerhalb von weniger als 1 Sekunde. Quenschen sind z.B. beschrieben in EP 1966291.

Das Produktgemisch (G) wird nach der Entnahme aus der Reaktionszone (R) des Pyrolysereaktos (P) abgekühlt, wodurch eine Kondensation der Styrol-Monomere und weiterer Komponenten stattfindet und ein kondensiertes Produktgemisch (K) enthaltend Styrol-Monomere und weitere Komponenten erhalten wird. Das Produktgemisch (G) wird auf eine Temperatur abgekühlt, die unter dem Kondensationspunkt von Styrol-Monomeren liegt. Bevorzugt wird das Produktgemisch (G) auf eine Temperatur unter 70 °C, besonders bevorzugt unter 50 °C, ganz besonders bevorzugt unter 40 °C gekühlt. In einer bevorzugten Ausführungsform wird das Produktgemisch (G) auf eine Temperatur von 0 °C bis 70 °C gekühlt. In einer besonders bevorzugten Ausführungsform wird das Produktgemisch (G) auf eine Temperatur von 0 °C bis 50 °C gekühlt. In einer ganz besonders bevorzugten Ausführungsform wird das Produktgemisch (G) auf eine Temperatur von 15 °C bis 40 °C gekühlt.

Die Abkühlung kann auf eine beliebige bekannte Art und Weise erfolgen. Beispielsweise ist die Abkühlung an einer festen Oberfläche möglich, die durch Wasser oder Luft gekühlt wird. Ebenfalls ist die Abkühlung durch einen Wassernebel möglich, der direkt mit dem Produktgemisch (G) in Kontakt gebracht wird. Bevorzugt findet die Abkühlung in einem Wassernebel statt.

Dabei werden die kondensierbaren Bestandteile des Produktgemischs (G) entsprechend ihren Dampfdrücken kondensiert und zusammen mit dem Wasser aufgefangen. Bei der Kondensation wird ein kondensiertes Produktgemisch (K) enthaltend Styrol-Monomere und weitere Bestandteile erhalten. Im Falle der Abkühlung in einem Wassernebel wird das kondensierte Produktgemisch (K) als zwei-Phasen-System mit dem Kühlwasser erhalten. In diesem Fall wird das kondensierte Produktgemisch (K) als aufschwimmende organische Phase von der wässrigen Phase getrennt. Die wässrige Phase kann erneut gekühlt und als Wassernebel zum Abkühlen des Produktgemischs (G) eingesetzt werden.

Das kondensierte Produktgemisch (K) enthält üblicherweise mehr als 50 Gew.-%, bezogen auf das Gesamtgewicht des kondensierten Produktgemischs (K), Styrol-Monomere und weniger als 50 Gew.-%, bezogen auf das Gesamtgewicht des kondensierten Produktgemischs (K), der weiteren Bestandteile. Vorzugsweise enthält das kondensierte Produktgemisch (K) 50 bis 99 Gew.-%, bezogen auf das Gesamtgewicht des kondensierten Produktgemischs (K), Styrol-Monomere und 1 bis 50 Gew.-%, bezogen auf das Gesamtgewicht des kondensierten Produktgemischs (K), der weiteren Bestandteile, besonders vorzugsweise 60 bis 99 Gew.-%, bezogen auf das Gesamtgewicht des kondensierten Produktgemischs (K), Styrol-Monomere und 1 bis 40 Gew.-%, bezogen auf das Gesamtgewicht des kondensierten Produktgemischs (K), der weiteren Bestandteile, ganz besonders vorzugsweise 75 bis 99 Gew.-%, bezogen auf das Gesamtgewicht des kondensierten Produktgemischs (K), Styrol-Monomere und 1 bis 25 Gew.-%, bezogen auf das Gesamtgewicht des kondensierten Produktgemischs (K), der weiteren Bestandteile.

Im weiteren Verlauf des Verfahrens wird das kondensierte Produktgemisch (K) in Styrol-Monomere und weitere Bestandteile getrennt. Diese Trennung kann auf beliebige bekannte Weise durchgeführt werden, die geeignet ist, Mischungen aus flüssigen Produkten und gegebenenfalls Feststoffen in die Bestandteile aufzutrennen. Abhängig von den weiteren Bestandteilen neben Styrol-Monomeren können geeignete Methoden zum Trennen des kondensierten Produktgemischs (K) in Styrol-Monomere und weitere Bestandteile beispielsweise Sedimentation, Zentrifugation, Filtration, Dekantieren, Destillation, Chromatographie, Kristallisation und Sublimation umfassen.

Sind in der kondensierten Produktmischung (K) Feststoffe enthalten, ist es vorteilhaft, vor der Trennung der flüssigen Bestandteile die Feststoffe abzutrennen. In diesem Fall ist es bevorzugt, den Feststoff durch Sedimentation, Zentrifugation, Filtration, Destillation, Sublimation und/oder Dekantieren, besonders bevorzugt durch Filtration, ganz besonders bevorzugt durch Filtration mittels Fest-Flüssig-Filter von den flüssigen Bestandteilen abzutrennen.

Die weitere Auftrennung der flüssigen Bestandteile in Styrol-Monomere und weitere Bestandteile umfasst vorzugsweise mindestens einen Schritt der Destillation, wie zum Beispiel fraktionierende Destillation, mindestens einen Schritt der Chromatographie, wie zum Beispiel Säulenchromatographie, HPLC oder Flash-Chromatographie, und/oder mindestens einen Schritt der Kristallisation, wie zum Beispiel fraktionierende Kristallisation. Besonders vorzugsweise umfasst die Auftrennung der flüssigen Bestandteile mindestens einen Schritt der Destillation, ganz besonders vorzugsweise mindestens einen Schritt der fraktionierenden Destillation. In einer ganz besonders bevorzugten Ausführungsform umfasst die Auftrennung der flüssigen Bestandteile in Styrol-Monomere und weitere Komponenten mindestens einen Schritt der fraktionierenden Destillation in einer oder mehreren Rektifikationskolonnen.

Häufig ist es vorteilhaft, zumindest einen Teil der weiteren Komponenten in die Reaktionszone (R) des Pyrolysereaktors (P) zurückzuführen. Dies ist insbesondere vorteilhaft, wenn ein Teil der weiteren Bestandteile Styrol-Oligomere wie z.B. Styrol-Dimere und Styrol-Trimere enthält. Dies ermöglicht eine insgesamt bessere Ausbeute an Styrol-Monomeren, da hierdurch auch die Depolymerisation der übrig gebliebenen Oligomere ermöglicht wird. Aus diesem Grund wird vorzugsweise zumindest ein Teil der weiteren Komponenten des kondensierten Produktgemischs (K), die von Styrol-Monomeren abgetrennt wurden, in die Reaktionszone (R) des Pyrolysereaktors (P) rückzuführen. Besonders bevorzugt ist hierbei eine kontinuierliche Rückführung der weiteren Komponenten.

In einer bevorzugten Ausführungsform bestehen die weiteren Komponenten, die in die Reaktionszone (R) des Pyrolysereaktors (P) zurückgeführt werden, im Wesentlichen aus Styrol-Oligomeren, vorzugsweise aus Styrol-Dimeren und Styrol-Trimeren. In diesem Zusammenhang bedeutet "im Wesentlichen", dass die weiteren Komponenten, die in die Reaktionszone (R) des Pyrolysereaktors (P) zurückgeführt werden, neben Styrol-Oligomeren keine weiteren Komponenten enthalten, die den Depolymerisationsprozess in der Reaktionszone (R) des Pyrolysereaktors (P) stören.

Ein weiterer Gegenstand der Erfindung ist die Verwendung eines Wirbelschichtreaktors zur Depolymerisation von Polystyrol bei einer Temperatur in der Reaktionszone (R) von 400 °C bis 1000 °C und einer mittleren Verweilzeit des Polystyrols in der Reaktionszone (R) von 0,01 s bis 10 s, wobei der Wirbelschichtreaktor in der Reaktionszone (R) eine Wirbelschicht aus Siliciumcarbid (SiC) umfasst. Besonders bevorzugt sind das Siliciumcarbid, die Temperatur in der Reaktionszone (R) und die mittlere Verweilzeit des Polystyrols in der Reaktionszone (R) so gewählt, wie für das erfindungsgemäße Verfahren beschrieben ist.

Außerdem ist Gegenstand der Erfindung auch eine Vorrichtung zur Durchführung des erfindungsgemäßen Verfahrens zur Herstellung von Styrol-Monomeren, bei der die Reaktionszone (R) und der Pyrolysereaktor (P) so gestaltet sind, dass eine schonende Depolymerisation von Polystyrol erfolgen kann, wobei die Vorrichtung als Pyrolysereaktor (P) einen Wirbelschichtreaktor umfassend eine Wirbelschicht aus Siliciumcarbid (SiC) in der Reaktionszone (R) umfasst, wobei das Siliciumcarbid bevorzugt eine gewichtsmittleren Partikelgröße von 20 bis 1000 µm, besonders bevorzugt 40 bis 500 µm aufweist, optional resistive Heizleiter an den Außenwänden der Reaktionszone (R), einen Verdampfer zum Erzeugen von Wasserdampf, einen Dampfüberhitzer zum Einstellen der gewünschten Dampftemperatur, eine Leitung zum Einführen des Dampfs in die Reaktionszone (R) sowie eine Quensche zum Abkühlen des Produktgemischs (G).

Die Erfindung wird durch die folgenden Beispiele, Figuren und Ansprüche illustriert.

### Beispiele

Aus einem inertisierten Vorlagebehälter (B101) wird das Polymer über eine volumetrische Dosierung in einen Gasjet dosiert, welcher die Partikel pneumatisch in die Reaktionszone eines Wirbelschichtreaktors (R101) mit SiC-Partikeln fördert. Die Heizenergie wird über resistive Heizleiter durch die metallische Reaktorwand in die Reaktionszone gebracht. Der Dampf für die Erzeugung der Wirbelschicht wird durch einen Verdampfer (D101) zur Verfügung gestellt und über einen Dampfüberhitzer (E102) auf die gewünschte Temperatur gebracht.

In der Reaktionszone wird mit dem Dampf die SiC-Wirbelschicht erzeugt und die pneumatisch eingeförderten Polymerpartikel in dem Wirbelbett vermischt und schließlich depolymerisiert.

Das Produktgemisch des Depolymerisations-Prozesses aus (R101) wird in der Kolonne (K101) in einem Wassernebel auf unter 50 °C gekühlt. Dabei werden die kondensierbaren Bestandteile entsprechend ihren Dampfdrücken kondensiert und mit dem Wasser im gekühlten Sumpf aufgefangen. Feststoffpartikel werden ebenfalls entsprechend ihrer Koaleszenz-Neigung mit dem Nebel niedergeschlagen. Aufschwimmende, nicht wasserlösliche Bestandteile werden über ein Skimmer-System in einen Pufferbehälter überführt. Von der aufkonzentrierten, organischen Fraktion des kondensierten Produktgemischs werden Proben abgefüllt. Diese werden mittels Gaschromatographie vermessen.

Das Wasser aus dem Kondensationsprozess wird über einen Wärmetauscher gekühlt und anschließend mit der Pumpe wieder in die Kühlkolonne (K101) geführt. Feststoffe werden gegebenenfalls über einen Fest-Flüssig-Filter abgetrennt.

### Beispiele 1.1 bis 1.6

Ein amorphes Polystyrol (GPPS) (INEOS Styrolution, Frankfurt) mit einer Schmelze-Volumenfließrate (Melt Volume Rate 200°/5 kg Belastung, ISO 1133) von etwa 28 cm³/10 min wurde in der oben beschriebenen Vorrichtung unter verschiedenen Bedingungen depolymerisiert. Hierbei wurde die Temperatur in der Reaktionszone des Wirbelschichtreaktors (R101) in dem Bereich 550 °C bis 650 °C und die Verweilzeit in dem Bereich von 0,46 bis 1 s variiert. Wenn nicht anders angegeben, wurde die Konzentration der Polymerzusammensetzung in der Reaktionszone bei 5 Gew.-% bezogen auf die Gesamtfüllung des Reaktors, bestehend aus SiC- und Polymerpartikeln, gehalten. Die Ausbeuten an Pyrolyse-Öl (Kondensat-Öl) und an Styrol-Monomeren abhängig von den Reaktionsbedingungen sind in der Tabelle 1 dargestellt. Die Zusammensetzungen des Pyrolyseöls sind in Tabelle 2 dargestellt.

### Beispiel 2

Beispiel 1.2 wurde mit einem schlagzähen amorphen Polystyrol (HIPS) (INEOS Styrolution, Frankfurt) mit einer Schmelze-Volumenfließrate (Melt Volume Rate 200 °C/5 kg Belastung, ISO 1133) von etwa 4 cm³/10 min wiederholt. Die Ausbeute an Styrol-Monomeren ist in der Tabelle 1 dargestellt. Die Zusammensetzung des Pyrolyseöls ist in Tabelle 2 dargestellt.

### Beispiel 3

Beispiel 1.1 wurde mit einer Polymerzusammensetzung bestehend aus 50 Gew.-% der Polymerzusammensetzung aus Beispiel 1 und 50 Gew.-% der Polymerzusammensetzung aus Beispiel 2 wiederholt. Die Ausbeute an Styrol-Monomeren ist in der Tabelle 1 dargestellt. Die Zusammensetzung des Pyrolyseöls ist in Tabelle 2 dargestellt.

**Tabelle 1: Ergebnisse der Pyrolyseversuche**

| Beispiel | Polymer | Temperatur in der Reaktionszone [° C] | Mittlere Verweilzeit in Reaktionszone [s] | Masseanteil des Kondensat-Öls bezogen auf eingesetzte Masse [Gew.-%] | Styrol-konzentration im Kondensat-Öl [Gew.-%] |
|---|---|---|---|---|---|
| 1.1 | GPPS | 550 | 0,54 | 70,6 | 74,8 |
| 1.2 | GPPS | 600 | 0,51 | 65,9 | 83,2 |
| 1.3 | GPPS | 550 | 0,46 | 64,8 | 68,2 |
| 1.4 | GPPS | 650 | 0,48 | 69,0 | 76,8 |
| 1.5 | GPPS | 550 | 0,66 | 72,3 | 76,3 |
| 1.6 | GPPS, 8 Gew.-% in Reaktionszone | 550 | 0,54 | 72,5 | 73,4 |
| 2 | HIPS | 600 | 0,51 | 45,7 | 70,9 |
| 3 | 50 Gew.-% GPPS / 50 Gew.-% HIPS | 550 | 0,54 | 91,9 | 70,1 |

**Tabelle 2: Zusammensetzung des Pyrolyseöls**

| Beispiel | Styrol-konzentration im Kondensat-Öl [Gew.-%] | Dimerkonzentration im Kondensat-Öl [Gew.-%] | Trimerkonzentration im Kondensat-Öl [Gew.-%] | Konzentration sonstiger Verbindungen im Kondensat-Öl [Gew.-%] |
|---|---|---|---|---|
| 1.1 | 74,8 | 8,4 | 8,3 | 8,6 |
| 1.2 | 83,2 | 6,0 | 0,6 | 10,3 |
| 1.3 | 68,2 | 10,7 | 9,8 | 11,2 |
| 1.4 | 76,8 | 4,5 | 0,7 | 17,9 |
| 1.5 | 76,3 | 7,0 | 5,3 | 11,3 |
| 1.6 | 73,4 | 8,2 | 1,1 | 17,3 |
| 2 | 70,9 | 7,3 | 3,3 | 18,6 |
| 3 | 70,1 | 6,6 | 7,5 | 15,7 |

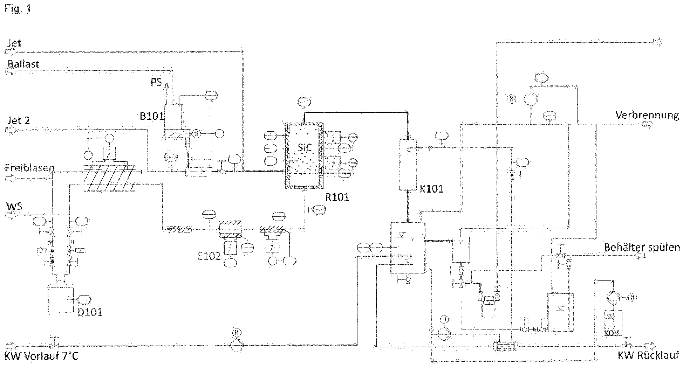

Erklärung der Figuren
Fig. 1 zeigt ein Fließbild der Technikums-Anlage zur Durchführung der Herstellung von Styrol-Monomeren durch Depolymerisation von Polystyrol.

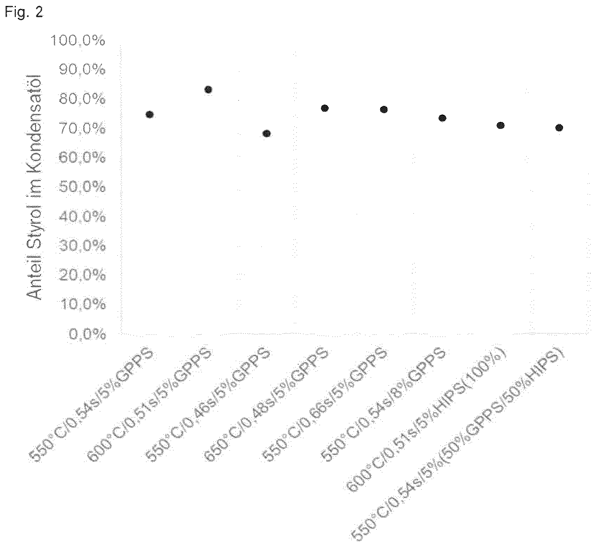

Fig. 2 zeigt eine Übersicht über die Styrol-Konzentration im Kondensat-Öl.

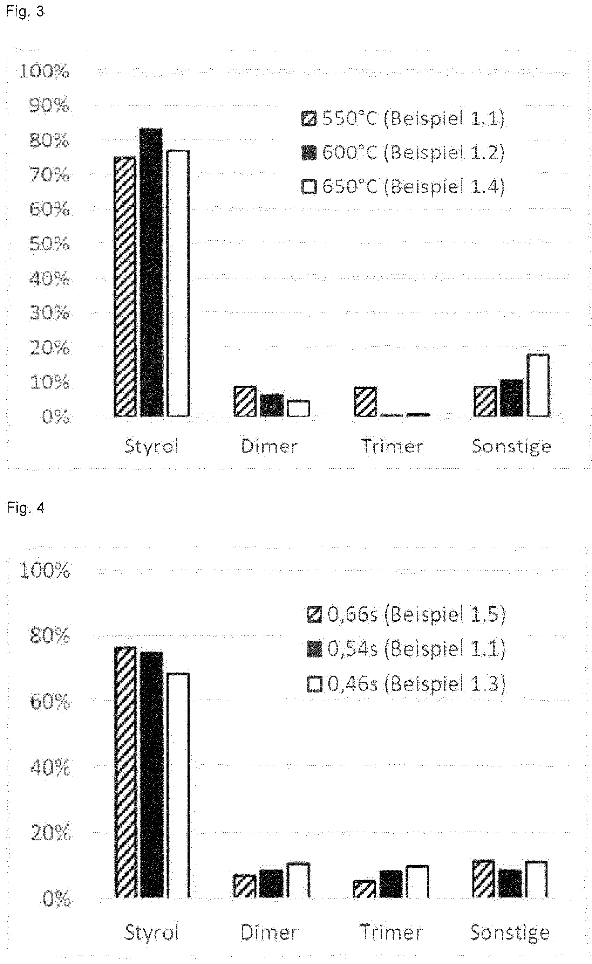

Fig. 3 zeigt die Abhängigkeit der Pyrolyse-Öl-Zusammensetzung von der Temperatur.
Fig. 4 zeigt die Abhängigkeit der Pyrolyse-Öl-Zusammensetzung von der Verweilzeit bei reinem GPPS.

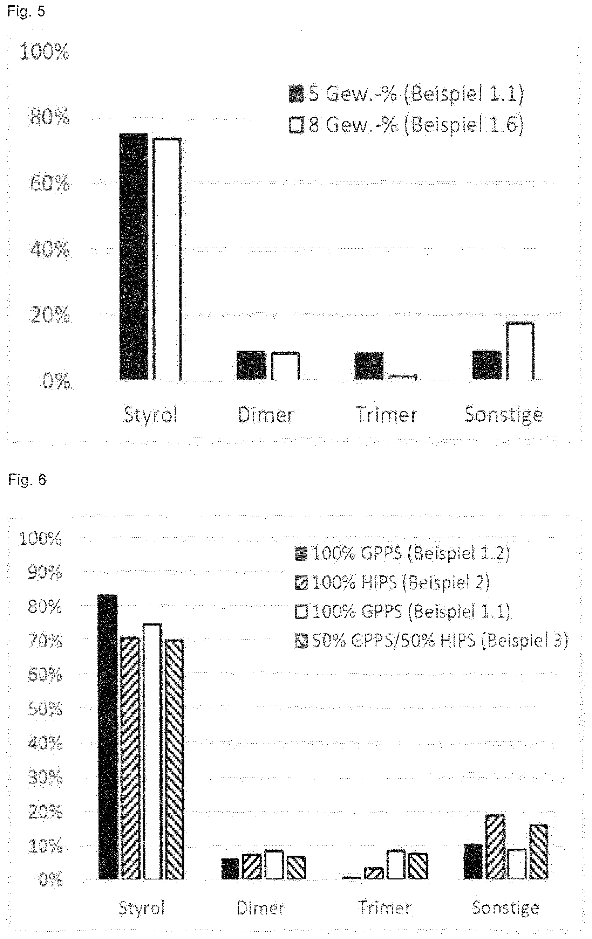

Fig. 5 zeigt die Abhängigkeit der Pyrolyse-Öl-Zusammensetzung von der Konzentration des Reaktionsgutes im Reaktor.
Fig. 6 zeigt die Abhängigkeit der Pyrolyse-Öl-Zusammensetzung von der Zusammensetzung des Reaktionsgutes.

## Patentansprüche

1. Verfahren zur Herstellung von Styrol-Monomeren durch Depolymerisation von Polystyrol, umfassend die Schritte:
a) Zuführen einer Polymerzusammensetzung (A) enthaltend 60 bis 99,9 Gew.-% Polystyrol, bezogen auf das Gesamtgewicht der Polymerzusammensetzung (A), in die Reaktionszone (R) eines Pyrolysereaktors (P);
b) thermisches Spalten des in der Polymerzusammensetzung (A) enthaltenen Polystyrols in der Reaktionszone (R) des Pyrolysereaktors (P) bei einer Temperatur von 400 °C bis 1000 °C, um ein Produktgemisch (G) enthaltend Styrol-Monomere und weitere Komponenten zu erhalten;
c) Entnehmen des in Schritt b) erhaltenen Produktgemisches (G) aus der Reaktionszone (R) des Pyrolysereaktors (P);
d) Abkühlen des in Schritt c) entnommenen Produktgemisches (G), um ein kondensiertes Produktgemisch (K) enthaltend Styrol-Monomere und weitere Komponenten zu erhalten; und
e) Abtrennen der Styrol-Monomere von den weiteren Komponenten des in Schritt d) erhaltenen kondensierten Produktgemisches (K),
wobei die mittlere Verweilzeit (Z) der Polymerzusammensetzung (A) in der Reaktionszone (R) des Pyrolysereaktors (P) von 0,01 s bis 10 s beträgt, und wobei der Pyrolysereaktor (P) ein Wirbelschichtreaktor ist, und wobei die Reaktionszone (R) des Wirbelschichtreaktors eine Siliciumcarbid-Wirbelschicht umfasst.

2. Verfahren zur Herstellung von Styrol-Monomeren durch Depolymerisation von Polystyrol, umfassend die Schritte:
a) Zuführen einer Polymerzusammensetzung (A) enthaltend 60 bis 99,9 Gew.-% Polystyrol, bezogen auf das Gesamtgewicht der Polymerzusammensetzung (A), in die Reaktionszone (R) eines Pyrolysereaktors (P);
b) thermisches Spalten des in der Polymerzusammensetzung (A) enthaltenen Polystyrols in der Reaktionszone (R) des Pyrolysereaktors (P) bei einer Temperatur von 400 °C bis 1000 °C, um ein Produktgemisch (G) enthaltend Styrol-Monomere und weitere Komponenten zu erhalten;
c) Entnehmen des in Schritt b) erhaltenen Produktgemisches (G) aus der Reaktionszone (R) des Pyrolysereaktors (P);
d) Abkühlen des in Schritt c) entnommenen Produktgemisches (G), um ein kondensiertes Produktgemisch (K) enthaltend Styrol-Monomere und weitere Komponenten zu erhalten; und
e) Abtrennen der Styrol-Monomere von den weiteren Komponenten des in Schritt d) erhaltenen kondensierten Produktgemisches (K), wobei die Temperatur in der Reaktionszone (R) des Pyrolysereaktors (P) 580 °C bis 630 °C beträgt und die mittlere Verweilzeit (Z) der Polymerzusammensetzung (A) in der Reaktionszone des Pyrolysereaktors 0,4 s bis 0,6 s beträgt.

3. Verfahren gemäß Anspruch 1 oder 2, wobei das in Schritt d) erhaltene kondensierte Produktgemisch (K) von 75 bis 99 Gew.-% Styrol und 1 bis 25 Gew.-% der weiteren Komponenten enthält, jeweils bezogen auf das Gesamtgewicht des Produktgemisches (K).

4. Verfahren gemäß Anspruch 2, wobei der Pyrolysereaktor (P) ein Wirbelschichtreaktor ist.

5. Verfahren gemäß Anspruch 4, wobei die Reaktionszone (R) des Wirbelschichtreaktors eine Siliciumcarbid-Wirbelschicht umfasst.

6. Verfahren gemäß einem der Ansprüche 1 bis 5, wobei zumindest ein Teil der in Schritt e) erhaltenen weiteren Komponenten in die Reaktionszone (R) des Pyrolysereaktors (P) rückgeführt wird.

7. Verfahren gemäß Anspruch 6, bei dem die im Schritt e) erhaltenen weiteren Komponenten, die in die Reaktionszone (R) des Pyrolysereaktors (P) rückgeführt werden, im Wesentlichen aus Styrol-Oligomeren bestehen.

8. Verfahren gemäß Anspruch 6 oder 7, bei dem die Rückführung der in Schritt e) erhaltenen weiteren Komponenten in die Reaktionszone (R) des Pyrolysereaktors (P) kontinuierlich durchgeführt wird.

9. Verfahren gemäß einem der Ansprüche 1 bis 8, bei dem die Polystyrol-Komponente in der Polymerzusammensetzung (A), die in Schritt a) in die Reaktionszone (R) des Pyrolysereaktors (R) zugeführt wird, im Wesentlichen aus GPPS (general purpose polystyrene) besteht.

10. Verfahren gemäß einem der Ansprüche 1 bis 9, bei dem in Schritt d) das in Schritt c) entnommene Produktgemisch (G) auf eine Temperatur von 0 °C bis 50 °C gekühlt wird.

11. Verfahren gemäß einem der Ansprüche 1 bis 10, bei dem die Polymerzusammensetzung (A) enthaltend Polystyrol in Schritt a) keine weiteren polymeren Komponenten enthält.

12. Verfahren gemäß einem der Ansprüche 1 bis 11, bei dem das Abtrennen der Styrol-Monomere in Schritt e) den Schritt der fraktionierenden Destillation umfasst.

13. Verwendung eines Wirbelschichtreaktors zur Depolymerisation von Polystyrol bei einer Temperatur in der Reaktionszone (R) von 400 °C bis 1000 °C und einer mittleren Verweilzeit des Polystyrols in der Reaktionszone (R) von 0,01 s bis 10 s, wobei der Wirbelschichtreaktor eine Siliciumcarbid-Wirbelschicht umfasst.

14. Vorrichtung zur Durchführung eines Verfahrens zur Herstellung von Styrol-Monomeren gemäß einem der Ansprüche 1 bis 12, bei der die Reaktionszone (R) und der Pyrolysereaktor (P) so gestaltet sind, dass eine schonende Depolymerisation von Polystyrol erfolgen kann, wobei der Pyrolysereaktor (P) ein Wirbelschichtreaktor ist, und wobei die Reaktionszone (R) des Wirbelschichtreaktors eine Siliciumcarbid-Wirbelschicht umfasst.

## Claims

1. Process for the preparation of styrene monomers by depolymerization of polystyrene, comprising the steps of:
(a) feeding a polymer composition (A) containing 60 to 99.9% by weight of polystyrene, based on the total weight of the polymer composition (A), into the reaction zone (R) of a pyrolysis reactor (P);
b) thermally cracking the polystyrene contained in the polymer composition (A) in the reaction zone (R) of the pyrolysis reactor (P) at a temperature of 400°C to 1000°C to obtain a product mixture (G) containing styrene monomers and further components;
c) removing the product mixture (G) obtained in step b) from the reaction zone (R) of the pyrolysis reactor (P);
d) cooling the product mixture (G) removed in step c) to obtain a condensed product mixture (K) containing styrene monomers and further components; and
e) separating the styrene monomers from the further components of the condensed product mixture (K) obtained in step d),
wherein the average residence time (Z) of the polymer composition (A) in the reaction zone (R) of the pyrolysis reactor (P) is from 0.01 s to 10 s, and wherein the pyrolysis reactor (P) is a fluidized bed reactor, and wherein the reaction zone (R) of the fluidized bed reactor comprises a silicon carbide fluidized bed.

2. Process for the preparation of styrene monomers by depolymerization of polystyrene, comprising the steps of:
(a) feeding a polymer composition (A) containing 60 to 99.9% by weight of polystyrene, based on the total weight of the polymer composition (A), into the reaction zone (R) of a pyrolysis reactor (P);
b) thermally cracking the polystyrene contained in the polymer composition (A) in the reaction zone (R) of the pyrolysis reactor (P) at a temperature of 400°C to 1000°C to obtain a product mixture (G) containing styrene monomers and further components;
c) removing the product mixture (G) obtained in step b) from the reaction zone (R) of the pyrolysis reactor (P);
d) cooling the product mixture (G) removed in step c) to obtain a condensed product mixture (K) containing styrene monomers and further components; and
e) separating the styrene monomers from the further components of the condensed product mixture (K) obtained in step d),
the temperature in the reaction zone (R) of the pyrolysis reactor (P) being from 580°C to 630°C and the average residence time (Z) of the polymer composition (A) in the reaction zone of the pyrolysis reactor being from 0.4 s to 0.6 s.

3. Process according to claim 1 or 2, wherein the condensed product mixture (K) obtained in step d) contains from 75 to 99% by weight of styrene and from 1 to 25% by weight of the further components, in each case based on the total weight of the product mixture (K).

4. Process according to claim 2, wherein the pyrolysis reactor (P) is a fluidized bed reactor.

5. Process according to claim 4, wherein the reaction zone (R) of the fluidized bed reactor comprises a silicon carbide fluidized bed.

6. Process according to any one of claims 1 to 5, wherein at least a part of the further components obtained in step e) is recycled to the reaction zone (R) of the pyrolysis reactor (P).

7. Process according to claim 6, in which the further components obtained in step e), which are recycled into the reaction zone (R) of the pyrolysis reactor (P), consist essentially of styrene oligomers.

8. Process according to claim 6 or 7, in which the recycling of the further components obtained in step e) into the reaction zone (R) of the pyrolysis reactor (P) is carried out continuously.

9. Process according to any one of claims 1 to 8, wherein the polystyrene component in the polymer composition (A) fed into the reaction zone (R) of the pyrolysis reactor (R) in step a) consists essentially of GPPS (general purpose polystyrene).

10. Process according to any one of claims 1 to 9, in which in step d) the product mixture (G) removed in step c) is cooled to a temperature of 0 °C to 50 °C.

11. Process according to any one of claims 1 to 10, wherein the polymer composition (A) comprising polystyrene in step a) contains no further polymeric components.

12. Process according to any one of claims 1 to 11, wherein the separation of the styrene monomers in step e) comprises the step of fractional distillation.

13. Use of a fluidized bed reactor for the depolymerization of polystyrene at a temperature in the reaction zone (R) of from 400°C to 1000°C and an average residence time of the polystyrene in the reaction zone (R) of from 0.01 s to 10 s, wherein the fluidized bed reactor comprises a silicon carbide fluidized bed.

14. Apparatus for carrying out a process for the production of styrene monomers according to any one of claims 1 to 12, wherein the reaction zone (R) and the pyrolysis reactor (P) are designed such that a gentle depolymerization of polystyrene can take place, wherein the pyrolysis reactor (P) is a fluidized bed reactor, and wherein the reaction zone (R) of the fluidized bed reactor comprises a silicon carbide fluidized bed.

## Revendications

1. Procédé de fabrication de monomères de styrène par dépolymérisation de polystyrène, comprenant les étapes suivantes :
a) amenée dans une zone de réaction (R) d'un réacteur de pyrolyse (P) d'une composition de polymères (A) contenant 60 à 99,9 % en poids de polystyrène, par rapport au poids total de la composition de polymères (A) ;
b) dissociation thermique du polystyrène contenu dans la composition de polymères (A) dans la zone de réaction (R) du réacteur de pyrolyse (P) à une température de 400 °C à 1 000 °C, pour obtenir un mélange de produits (G) contenant des monomères de styrène et d'autres composants ;
c) prélèvement, de la zone de réaction (R) du réacteur de pyrolyse (P), du mélange de produits (G) obtenu dans l'étape b) ;
d) refroidissement du mélange de produits (G) prélevé dans l'étape c) pour obtenir un mélange de produits condensé (K) contenant des monomères de styrène et d'autres composants ; et
e) séparation des monomères de styrène des autres composants du mélange de produits condensé (K) obtenu dans l'étape d),
dans lequel le temps de séjour moyen (Z) de la composition de polymères (A) dans la zone de réaction (R) du réacteur de pyrolyse (P) est de 0,01 s à 10 s, et le réacteur de pyrolyse (P) est un réacteur à lit fluidisé, et la zone de réaction (R) du réacteur à lit fluidisé comprend un lit fluidisé de carbure de silicium.

2. Procédé de fabrication de monomères de styrène par dépolymérisation de polystyrène, comprenant les étapes suivantes :
a) amenée à la zone de réaction (R) d'un réacteur de pyrolyse (P) d'une composition de polymères (A) contenant 60 à 99,9 % en poids de polystyrène, par rapport au poids total de la composition de polymères (A) ;
b) dissociation thermique du polystyrène contenu dans la composition de polymères (A) dans la zone de réaction (R) du réacteur de pyrolyse (P) à une température de 400 °C à 1 000 °C, pour obtenir un mélange de produits (G) contenant des monomères de styrène et d'autres composants ;
c) prélèvement, de la zone de réaction (R) du réacteur de pyrolyse (P), du mélange de produits (G) obtenu dans l'étape b) ;
d) refroidissement du mélange de produits (G) prélevé dans l'étape c), pour obtenir un mélange de produits condensé (K) contenant des monomères de styrène et d'autres composants ; et
e) séparation des monomères de styrène des autres composants du mélange de produits condensé (K) obtenu dans l'étape d), la température dans la zone de réaction (R) du réacteur de pyrolyse (P) étant de 580 °C à 630 °C et le temps de séjour moyen (Z) de la composition de polymères (A) dans la zone de réaction du réacteur de pyrolyse étant de 0,4 s à 0,6 s.

3. Procédé selon la revendication 1 ou 2, dans lequel le mélange de produits condensé (K) obtenu dans l'étape d) contient de 75 à 99 % en poids de styrène et de 1 à 25 % en poids des autres composants, dans chaque cas par rapport au poids total du mélange de produits (K).

4. Procédé selon la revendication 2, dans lequel le réacteur de pyrolyse (P) est un réacteur à lit fluidisé.

5. Procédé selon la revendication 4, dans lequel la zone de réaction (R) du réacteur à lit fluidisé comprend un lit fluidisé de carbure de silicium.

6. Procédé selon l'une des revendications 1 à 5, dans lequel au moins une partie des autres composants obtenus dans l'étape e) est renvoyée dans la zone de réaction (R) du réacteur de pyrolyse (P).

7. Procédé selon la revendication 6, dans lequel les autres composants obtenus dans l'étape e), qui sont renvoyés dans la zone de réaction (R) du réacteur de pyrolyse (P), sont pour l'essentiel constitués d'oligomères de styrène.

8. Procédé selon la revendication 6 ou 7, dans lequel le renvoi des autres composants obtenus dans l'étape e) dans la zone de réaction (R) du réacteur de pyrolyse (P) est mis en œuvre en continu.

9. Procédé selon l'une des revendications 1 à 8, dans lequel le composant polystyrène dans la composition de polymères (A) qui est envoyée dans l'étape a) dans la zone de réaction (R) du réacteur de pyrolyse (P) est pour l'essentiel constitué de GPPS (general purpose polystyrène), polystyrène à usage général.

10. Procédé selon l'une des revendications 1 à 9, dans lequel dans l'étape d), le mélange de produits (G) prélevé dans l'étape c) est refroidi à une température de 0 °C à 50 °C.

11. Procédé selon l'une des revendications 1 à 10, dans lequel la composition de polymères (A) contenant du polystyrène dans l'étape a) ne contient aucun autre composant polymère.

12. Procédé selon l'une des revendications 1 à 11, dans lequel la séparation des monomères de styrène dans l'étape e) comprend l'étape de distillation fractionnée.

13. Utilisation d'un réacteur à lit fluidisé pour la dépolymérisation de polystyrène à une température dans la zone de réaction (R) de 400 °C à 1 000 °C et pour un temps de séjour moyen du polystyrène dans la zone de réaction (R) de 0,01 s à 10 s, le réacteur à lit fluidisé comprenant un lit fluidisé de carbure de silicium.

14. Dispositif pour la mise en œuvre d'un procédé de fabrication de monomères de styrène selon l'une des revendications 1 à 12, dans lequel la zone de réaction (R) et le réacteur de pyrolyse (P) sont agencés de façon qu'une dépolymérisation ménagée du polystyrène puisse avoir lieu, le réacteur de pyrolyse (P) étant un réacteur à lit fluidisé, et la zone de réaction (R) du réacteur à lit fluidisé comprenant un lit fluidisé de carbure de silicium.
